# EUROPÄISCHE PATENTANMELDUNG

(11) **EP 1 992 683 A1**
(43) Veröffentlichungstag der Anmeldung: **19.11.2008**
(21) Anmeldenummer: 07009784.5
(22) Anmeldetag: 16.05.2007
(51) Int. Cl.: C12M 1/08, C12M 1/113, C12M 1/107

(54) **Biogasanlage zur Herstellung von Biogas in einem einstufigen Durchflussverfahren**

(71) Anmelder: UTS Biogastechnik GmbH, 84419 Obertaufkirchen (DE)
(72) Erfinder: Bürger, Adam, 83527 Haag (DE)
(74) Vertreter: Liebl, Thomas

(57) **Zusammenfassung**

Die Erfindung betrifft eine Biogasanlage zur Herstellung von Biogas durch anaerobe Nassvergärung eines Biomasse enthaltenden Substrats in einem einstufigen Durchflussverfahren, mit wenigstens zwei Fermenterbehältern (2, 3, 4) insbesondere mit wenigstens einem Hauptfermenter, wenigstens einem Nachfermenter und wenigstens einem Endlager. Erfindungsgemäß besteht jeder der Fermenterbehälter (2, 3, 4) in der Art einer langgestreckten, übedachten Fermenterwanne aus einer Bodenwand und aus zwei parallelen, beabstandeten, vertikalen und geraden Längsseitenwänden (5 bis 8), die endseitig an der Breitseite der Fermenterbehälter (2, 3, 4) durch vertikale, konvex ausgeformte Breitseitenwände (9 bis 14) verbunden sind. Zudem grenzen wenigstens zwei Fermenterbehälter (2, 3, 4) in einem Fermenterverbund (1) mit Längsseitenbereichen zumindest teilweise nebeneinanderliegend aneinander und sind dort durch einen gemeinsamen Längsseitenwandbereich als Verbundwand (6, 7) verbunden.

## Beschreibung

Die Erfindung betrifft eine Biogasanlage zur Herstellung von Biogas durch anaerobe Nassvergärung eines Biomasse enthaltenden Substrats in einem einstufigen Durchflussverfahren nach dem Oberbegriff des Anspruchs 1.

Die Biogasgewinnung zur Energieerzeugung gewinnt vorrangig im landwirtschaftlichen Bereich zunehmend an Bedeutung. Die Gründe dafür sind im Wesentlichen die Erschließung neuer Einkommensquellen in der Landwirtschaft durch eine staatlich geförderte regenerative und umweltverträgliche Energiegewinnung. Die dafür bekannten Biogasanlagen sind meist für einen kontinuierlichen einstufigen Betrieb ausgelegt mit wenigstens einem Fermenterbehälter, in dem in einem anaeroben Nassvergärungsprozess zur Gewinnung von Biogas Biomasse vergoren werden. Regelmäßig sind weitere Fermenterbehälter Bestandteil der Biogasanlage, insbesondere zusätzlich wenigstens ein Nachfermenterbehälter und wenigstens ein Endlagerbehälter. Zur Beschickung der Biogasanlage mit vergärbaren Stoffen ist zudem meist eine automatisierte Zudosiereinrichtung vorgesehen, wobei für flüssige Substratbestandteile Pumpen und für feste Substratbestandteile Biomassenfeststoff-Zudosiereinrichtungen gegebenenfalls mit Wägeeinrichtungen und Befüllschnecken oder Förderbändern verwendet werden. Zudem sind in den Behältern und Fermentern Rühreinrichtungen für eine gleichmäßige Verteilung der Substratbestandteile angebracht (EP 1 251 165 A1) um einerseits den Wirkungsgrad des Fermentationsprozesses zu erhöhen und andererseits der Bildung von Schwimmschichten und Sinkschichten entgegenzuwirken.

Für kontinuierlich in einem einstufigen Durchflussverfahren arbeitende Biogasanlagen werden üblicherweise zylindrisch aufgebaute Fermenterbehälter in grundsätzlich zwei unterschiedlichen Anordnungen verwendet:

In einer ersten Anordnung besteht der Fermenterbehälter aus einem zylinderrohrförmigen liegenden Tank mit einer horizontalen Zylinderachse und mit einer zentralen, horizontal liegenden Rührwelle, an der axial und radial zueinander versetzt eine Mehrzahl von Rührpaddeln angeordnet sind (DE 199 28 212 A1). Ein solcher liegender Tank mit einer Paddelrühreinrichtung kann mit vertretbaren Mitteln nicht beliebig groß gestaltet werden, so dass entsprechende Fermenterbehälter zweckmäßig nur in begrenzten Größenordnung projektiert werden. Nachteilig sind Reparaturen und Wartungsarbeiten an den Paddelrühreinrichtungen nur mit erheblichem Aufwand bei entleertem Fermenterbehälter möglich. Die Herstellung einer großen Biogasanlage mit mehreren solchen liegenden Zylindertanks erfordert viel Grundstückfläche und Platz, da zwischen den liegenden Zylindertanks viel ungenützter Platz verbleibt.

Bei einer grundsätzlich anderen zweiten Ausführungsform werden zylindrische Fermenterbehälter mit vertikaler Zylinderachse meist als Hochbehälter oder teilweise im Erdboden versenkte Behälter aus Beton oder Stahl eingesetzt (EP 1 130 084 B1; DE 200 08 186 U1), die in allgemein bekannter Weise durch eine Betondecke, eine Stahldecke oder durch ein ein- und zweilagiges Foliendach, welches gegebenenfalls einen Gasspeicher bildet, gasdicht abgedeckt sind. Bei solchen zylindrischen Fermenterbehältern werden regelmäßig Rühreinrichtungen in der Art von höhenverstellbaren Tauchrührgeräten oder seitlich durch eine Behälterwand eingeführten Rührgeräten verwendet. Auch hier ergeben sich bei nebeneinander angeordneten zylindrischen Behältern, beispielsweise einem Hauptfermenter, einem Nachfermenter und einem Endlager eine unzureichende Grundflächenausnützung durch rundungsbedingt schlecht nutzbare Freiräume zwischen dem Behältern.

Der Trend geht zu immer größeren Biogasanlagen, wobei eine größere Anzahl von Zylinderbehältern verwendet wird, wodurch der vorstehend erläuterte Nachteil einer unzureichenden Grundflächenausnützung weiter verstärkt wird. Alternativ dazu können für größere Biogasanlagen weniger Behälter jedoch mit sehr großen Behälterdurchmessern eingesetzt werden, was jedoch zu einem bautechnisch hohen Aufwand für so große Zylinderbehälter führt. Zudem ist auch hier die Grundflächenausnützung nicht optimal und eine Einpassung solcher großer Zylinderbehälter in vorhandene Geländestrukturen und Grundstückseigentumsverhältnisse kann zu Schwierigkeiten führen.

Weiter ist das Rühren und effektive Durchmischen des Substrats mit Rühreinrichtungen, beispielsweise mit Tauchrührgeräten in den vorstehenden Zylinderbehältern grundsätzlich schwierig: Bei einer gleichmäßig umlaufenden Substratbewegung im Zylinderbehälter erfolgt im Bereich der Zylinderachse praktisch keine Substratbewegung oder Substratdurchmischung. Um dem entgegenzuwirken wird in aufwändiger Weise die Höhenstellung und Winkelstellung der Tauchrührgeräte in Zeitabständen verändert. Zudem erfordert die ständige Strömungsumlenkung an den Zylinderwänden einen relativ hohen Energieaufwand beim Rühr- und Durchmischungsvorgang.

Aufgabe der Erfindung ist es, demgegenüber eine Biogasanlage vorzuschlagen, bei der ein sehr kompakter Aufbau bei verbesserter Grundflächenausnützung und kurzen Leitungsverbindungen möglich ist und bei der zudem zur Vermeidung von Schwimm- und Sinkschichten ein effektiver und energiesparender Rührvorgang durchgeführt werden kann.

Diese Aufgabe wird mit den Merkmalen des Anspruchs 1 gelöst.

Gemäß Anspruch 1 besteht jeder der wenigstens zwei Fermenterbehälter in der Art einer langgestreckten überdachten Fermenterwanne aus einer Bodenwand und aus zwei parallelen beabstandeten vertikalen und geraden Längsseitenwände, die endseitig an der Breitseite des Fermenterbehälters durch vertikale konvex ausgeformte Breitseitenwände verbunden sind. Die wenigstens zwei Fermenterbehälter grenzen in einem Fermenterverbund mit Längsseitenbereichen zumindest teilweise nebeneinanderliegend aneinander und sind dort durch einen gemeinsamen Längsseitenwandbereich als Verbundwand miteinander verbunden. Bei mehr als zwei Fermenterbehältern können diese entsprechend aneinandergereiht werden, wobei an den aneinandergrenzenden geraden Längsseitenbereichen jeweils Verbundwände gebildet sind.

Dadurch ergibt sich ein sehr kompakter Aufbau, insbesondere bei großen Biogasanlagen mit einer optimalen Bodenflächenausnützung und reduzierten Behälterbaukosten durch die Verwendung gemeinsamer Verbundwände für jeweils zwei dort aneinandergrenzende Behälter. Durch die an den Längsenden konvex ausgeformten Breitseitenwänden kann in den Behältern eine für den Rühr- und Durchmischungsvorgang günstige umlaufende Strömung erzeugt werden, welche im Fermenterbehälter in den Längsseitenbereichen gegenläufig sein kann und in den konvex ausgeformten Breitseitenbereichen umgelenkt wird - reine Rechteckbehälter sind dagegen insbesondere wegen rührtechnisch unzureichend erfassbarer Eckräume ungeeignet.

In einer besonders bevorzugten Weiterbildung nach Anspruch 2 sind in den Fermenterbehältern in der Längsmitte verlaufende, vertikale Mittenwände angeordnet, die mit ihren Mittenwandlängsenden beabstandet zu den benachbarten konvex ausgeformten Breitseitenwänden liegen. Dadurch sind in den Fermenterbehältern jeweils zwei parallel verlaufende, langgestreckte gegensinnig durchströmbare Längskanäle mit jeweils endseitigen Kanalumlenkungen durch die Breitseitenwände zu einer Endlos-Kanalstruktur gebildet. Eine solche Endlos-Kanalstruktur ist rührtechnisch mit relativ wenig Energieaufwand und effektiven Rührergebnissen sehr gut zu betreiben.

In einer dazu konkreten vorteilhaften Ausführung nach Anspruch 3 sind die Breitseitenwände halbkreisförmig kontinuierlich gebogene oder durch gerade Teilstücke bogenförmig zusammengesetzte Bogenwände. Zudem ist der Längsabstand der Mittenwandlängsenden zu den benachbarten Bogenwänden größer oder zumindest gleich der Quererstreckung eines Längskanals. Mit diesen Maßnahmen wird ein gleichmäßiger Strömungsverlauf begünstigt.

Grundsätzlich können die Fermenterbehälter in allen an sich bekannten Bauweisen beispielsweise als Monolith, aus Stahl, aus Holz, etc. hergestellt werden. Eine besonders kostengünstige und schnelle Bauweise wird jedoch mit den Merkmalen des Anspruchs 4 erreicht: Dabei sind die Längsseitenwände und/oder die Breitseitenwände zumindest teilweise aus Fertigteilelementen zusammengesetzt. Insbesondere werden Fertigteilelemente für die Übergangsbereiche zwischen einer gemeinsamen Verbundwand und anschließenden einem einzelnen Fermenterbehälter zugeordneten Wand vorgeschlagen, da diese Übergangsbereiche vor Ort individuell nur aufwändig, beispielsweise in Verbindung mit einer komplizierten Betonschalung, herstellbar sind. Wenn dagegen diese Übergangsbereiche als Fertigteilelemente vorliegen, können beispielsweise die geraden Längswände in einer gewünschten Länge einfach vor Ort, beispielsweise durch eine einfache gerade Betonschalung hergestellt werden. Ebenso können die Bogenwände durch einfach hergestellte gerade Teilstücke individuell in der gewünschten Ausdehnung zusammengesetzt werden, wobei auch gerade verlaufende oder bogenförmige Wandelemente als Fertigteile zur Verfügung gestellt werden können. Dadurch ergibt sich ein Baukastensystem mit dem kostengünstig und schnell ein Fermenterverbund mit vorgebbaren Standartgrößen herstellbar ist, jedoch auch individuelle Änderungen und Anpassungen in den Größenverhältnissen einfach möglich sind.

Gemäß Anspruch 5 sind die einzelnen Behälterwände vorzugsweise aus Beton hergestellt, wobei jedoch auch andere Materialien, wie Stahl, Kunststoff oder Holz einzeln oder in einem Verbund eingesetzt werden können. Wesentlich ist dabei, dass zumindest die Außenwände des Fermenterverbunds statisch sicher ausgebildet sind. Die gemeinsame Verbundwand zweier angrenzender Behälter ist bei gleicher Befüllung der Behälter dagegen nur wenig belastet und kann somit unter Beachtung eines stets im Wesentlichen gleichen Füllgrads gegebenenfalls schwächer dimensioniert werden. Solche Zwischenwände und insbesondere die nur wenig belasteten Mittenwände als Kanaltrennwände können somit nach Anspruch 6 auch als Tauchwände in der Art gespannter Textilfahnen und/oder Kunststoffbahnen und/oder Holzwände ausgeführt werden, was zu einer weiteren Kostenreduzierung für den Behälterbau des Fermenterverbunds führt.

Wenn als Behälterabdeckung eine Betondecke vorgesehen ist, kann diese den Druck nach außen auf verbundäußere Wände für eine sichere Statik aufnehmen. Bei Behälterausführungen ohne Betondecke, insbesondere mit Foliendächern wird dagegen mit Anspruch 7 vorgeschlagen, dass die Längsseitenwände eines Fermenterbehälters jedoch zumindest die äußeren Längsseitenwände des Fermenterverbunds im oberen Bereich durch Kreuzstreben zur statischen Aussteifung verbunden sind. Die Kreuzstreben können auch als leiterförmige Brückenstege ausgeführt sein, welche begehbar und zur Halterung von Technikelementen, beispielsweise für Rührwerke, ausgeführt sein können.

Je nach den Gegebenheiten können der Fermenterverbund gemeinsam oder einzelne Fermenterbehälter separat durch eine Betondecke und/oder durch Stahlplatten und/oder durch ein Foliendach oder durch Kombinationen daraus gasdicht abgedeckt sein, wobei ein Foliendach zudem als Gasspeicher genutzt werden kann.

Eine bevorzugte Kombination besteht nach Anspruch 9 darin, dass die Bereiche in den Längsseitenwandbereichen durch eine Betondecke und die endseitigen Bogenwandbereiche durch Foliendächer abgedeckt werden, wobei die Betondecke in statisch kritischen Längsseitenwandbereich die statische Aussteifung übernimmt.

Auch beim erfindungsgemäßen Fermenterverbund können nach Anspruch 10 in den Betondecken und/oder Foliendachbereichen an sich bekannte Revisionsöffnungen, insbesondere Revisionsschächte und/oder Sichtfenster angeordnet werden.

Für den erforderlichen Rühr- und Durchmischungsvorgang wird mit Anspruch 11 vorgeschlagen, in den Fermenterbehältern, vorzugsweise in den Längskanälen Rühreinrichtungen anzubringen, womit das Substrat in eine umlaufende Bewegung versetzbar ist.

Dazu werden mit Anspruch 12 an sich bekannte Tauchrührgeräte oder seitlich durch eine Außenwand des Fermenterverbunds einführbare Rührwerke vorgeschlagen. Zudem wird in einer bevorzugten Ausführung ein Rührwerk als Paddelrührwerk mit vertikaler Rührwelle vorgeschlagen, wobei die Rührwelle im Bereich einer Mittenwand insbesondere in deren Längsmitte angeordnet ist. Dadurch drehen die Paddel mit einer Schubrichtung in einem Längskanal und mit der gegensinnigen Schubrichtung im anderen parallelen Längskanal. Die vorstehend beschriebenen Rühreinrichtungen können jeweils einzeln verwendet werden oder gegebenenfalls auch kombiniert werden.

Bei einem Fermenterverbund mit mehreren Fermenterbehältern wird nach Anspruch 13 mit wenigstens einem Fermenterbehälter mit einer vergleichsweise geringeren Längserstreckung im Fermenterverbund ein Freiraum geschaffen, in dem ein Technikgebäude, insbesondere für Pumpen und/oder für Steuerungen und/oder für ein Blockheizkraftwerk oder einen Gasspeicherraum kompakt mit vorteilhaft kurzen Leitungswegen integrierbar ist.

Gemäß Anspruch 14 kann auch am erfindungsgemäßen Fermenterverbund wenigstens eine an sich bekannte Zudosieranlage für flüssige und/oder feste Substratbestandteile angeordnet werden. Zudem können Leitungsverbindungen zwischen den Fermenterbehältern, insbesondere Überläufe, jeweils unmittelbar im Bereich einer Verbundwand angebracht werden. Dadurch können aufwändig lange Leitungsverbindungen und gegebenenfalls dadurch erforderliche Pumpenanordnungen eingespart werden.

Anhand einer Zeichnung werden Ausführungsbeispiele der Erfindung weiter erläutert.

Es zeigen:
- Fig. 1: eine Draufsicht auf einen Fermenterverbund aus drei Fermenterbehältern (ohne Abdeckung),
- Fig. 2: eine Draufsicht entsprechend Fig. 1 mit einer statischen Aussteifung durch Kreuzstreben,
- Fig. 3: eine Draufsicht entsprechend Fig. 1 mit Paddelrührwerken in den Fermenterbehältern, und
- Fig. 4: eine Draufsicht entsprechend Fig. 1 mit einer Kombination unterschiedlicher Abdeckungen und einem Technikgebäude.

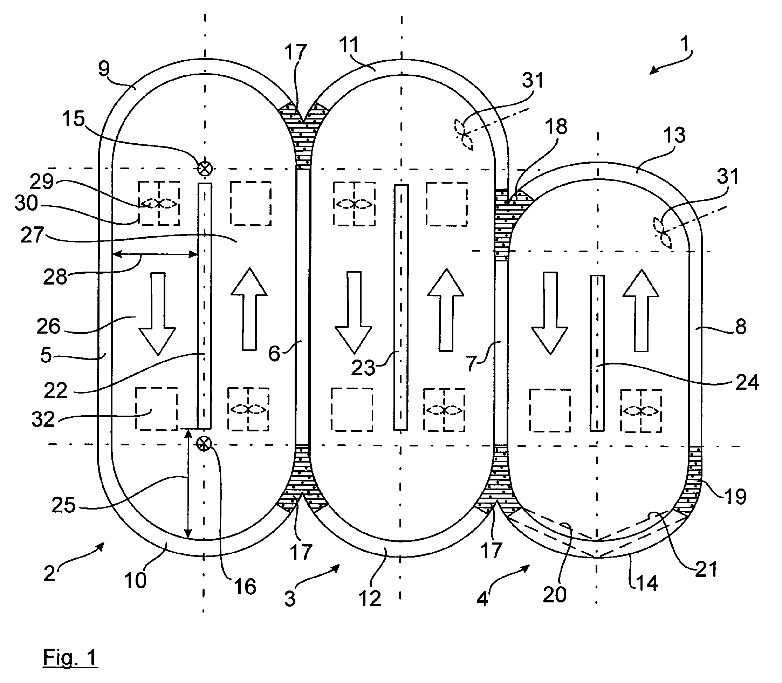

In Fig. 1 ist eine Draufsicht auf einen Fermenterverbund 1 einer Biogasanlage gezeigt, der aus drei Fermenterbehältern 2, 3, 4 besteht. An sich bekannte weitere Bauteile der Biogasanlage, insbesondere eine Zudosiereinrichtung sowie die gasdichte Abdeckung des Fermenterverbunds 1 sind wegen der besseren Übersichtlichkeit weggelassen.

Alle drei Fermenterbehälter 2, 3, 4 sind jeweils als langgestreckte Fermenterwannen ausgeführt, mit jeweils gegenüberliegenden geraden vertikalen und parallelen Längsseitenwänden 5, 6, 7, 8. Dabei haben die nebeneinanderliegenden Fermenterbehälter 2 und 3 eine gemeinsame Längsseitenwand als Verbundwand 6. Ebenso haben die aneinandergrenzenden Fermenterbehälter 3 und 4 eine gemeinsame Längsseitenwand als Verbundwand 7. Wegen der kürzeren Längserstreckung des Fermenterbehälters 4 ist die gemeinsame Verbundwand 7 etwas kürzer und erstreckt sich als gerader Wandteil noch in der Längsseitenwand des Fermenterbehälters 3.

An die einem Fermenterbehälter 2, 3, 4 zugeordneten gegenüberliegenden Verbundwände bzw. Längsseitenwände 5, 6, 7, 8 schließen sich jeweils halbkreisförmig gebogene verbundäußere vertikale Bogenwände 9, 10, 11, 12, 13, 14 an. Die halbkreisförmigen Bogenwände 9, 10, 11, 12, 13, 14 haben jeweils einen Kreismittelpunkt 15, 16 (nur im Fermenterbehälter 2 eingezeichnet), so dass sich die Längsseitenwände mit gerade Flucht jeweils im Bereich zwischen den durch die Kreismittelpunkte 6, 7 eingezeichneten strichpunktierten Querlinien erstrecken.

Zur Herstellung der Wände der Fermenterbehälter 2, 3, 4 sind strichliert hervorgehobene Fertigteile 17, 18, 19 verwendet, wobei die Fertigteile 17, 18 jeweils am Übergangsbereich zwischen einer gemeinsamen Verbundwand 6, 7 und einer anschließenden, einem Einzelfermenter zugeordneten Wand eingesetzt sind. Diese Bereiche wären bei einer individuellen Einschalung des hier gezeigten Betonwandbehälters schwierig herzustellen. Die an die Fertigteile 17, 18 anschließenden Wandbereiche sind dagegen relativ einfach vor Ort mit üblichen Schalungsteilen herstellbar, wobei damit auch individuelle Längen und Breiten der Behälter einfach zu gestalten sind. Mit dem Fertigteil 19 ist auch angedeutet, dass die Bogenwände 9 bis 14 aus Fertigteilelementen zusammengesetzt werden können. Zudem ist strichliert angedeutet, dass beispielsweise die Bogenwand 14 auch aus geraden Teilstücken 20, 21 zusammengesetzt sein könnte.

Weiter ist jeweils eine Mittenwand 22, 23, 24 in jedem der Fermenterbehälter 2, 3, 4 errichtet, die vertikal in der Längsmitte verläuft und mit deren Mittenwandlängsenden in einem Mittenwandlängsabstand (entsprechend Pfeil 25) von der in Längsrichtung gesehenen Innenseite der jeweiligen Bogenwand 10 liegt.

Durch die wannenförmigen Fermenterbehälter 2, 3, 4 in Verbindung mit deren Mittenwände 22, 23, 24 werden jeweils zwei langgestreckte gegensinnig durchstörmbare Längskanäle 26, 27 ausgebildet (nur anhand des Fermenterbehälters 2 erläutert). Die Quererstreckung entsprechend Pfeil 28 der Längskanäle 26, 27 ist jeweils geringer als der Mittenwandlängsabstand 25, wodurch Kanalumlenkbögen in den jeweiligen Endseiten der Längskanäle 26, 27 durch die Bogenwände 9, 10 ausgebildet sind. Dadurch können in den Längskanälen 26, 27 gegensinnige Substratströmungen (schematisch durch die großen Strömungspfeile dargestellt) hergestellt werden, was unter Einbeziehung der Umlenkung an den Bogenwänden 9, 10 zu einer endlos umlaufenden Strömung führt.

Zur Herstellung der umlaufenden Substratströmung sind in den Fermenterbehältern 2, 3, 4 jeweils schematisch eingezeichnete, an sich bekannte höhenverstellbare Tauchrührgeräte 29 verwendet, welche durch Serviceschächte 30 eingesetzt sind. Die Tauchrührgeräte 29 sind hier strömungseffektiv jeweils im Bereich der Längsströmung in den Längskanälen 26, 27 angeordnet. Weiter können die schematisch eingezeichneten weiteren Rührwerke 31 verwendet werden, welche seitlich durch eine Außenwand, hier durch die Bogenwände 11, 13 in die Fermenterbehälter 3, 4 eingeführt sind. Zudem ist die Möglichkeit von Sichtfenstern 32 in einer Beton- oder gegebenenfalls Stahlabdeckung angedeutet.

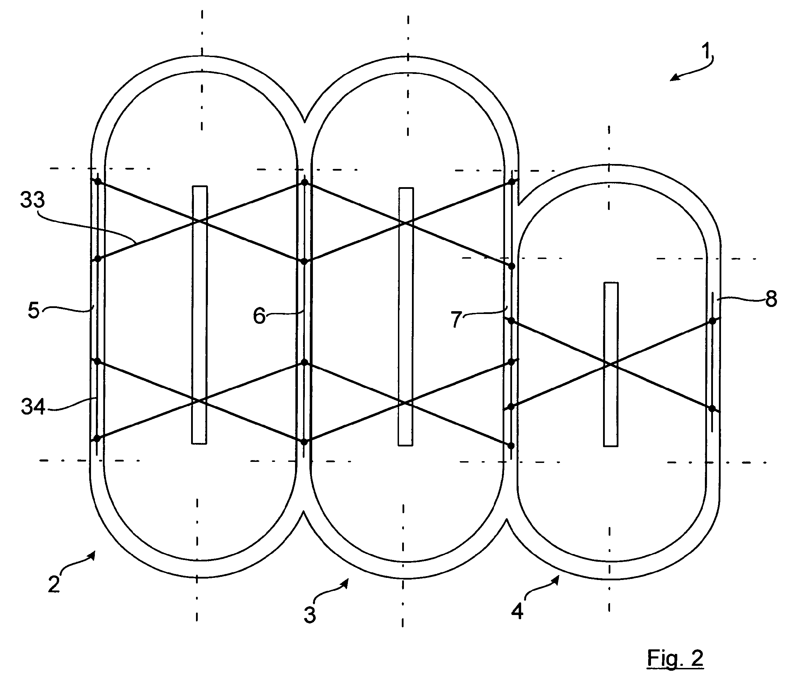

In Fig. 2 ist ein der Fig. 1 entsprechender Fermenterverbund 1 aus den Fermenterbehältern 2, 3, 4 dargestellt, der mit einem Foliendach abgedeckt werden soll. Ersichtlich sind bei befüllten Fermenterbehältern 2, 3, 4 insbesondere die Längsseitenwände 5, 6, 7, 8, in erster Linie die äußeren Längsseitenwände 5 und 8 stark zur Außenseite hin druckbelastet. Um hier die erforderliche Statik zu gewährleisten, können die Fermenterbehälter 2, 3, 4 in den Erdboden eingelassen werden oder bei einem Hochbehälter mit einer Betondecke abgedeckt werden, die den erforderlichen Zusammenhalt herstellt. Ein Foliendach, wie es im Fermenterverbund 1 der Fig. 2 als Abdeckung angebracht werden soll, kann einen solchen Zusammenhalt der Längsseitenwände 5, 6, 7, 8 nicht erbringen, so dass hier jeweils im oberen Bereich der Längsseitenwände 5, 6, 7, 8 Kreuzstrebenanordnungen 33 angeordnet sind, welche mit in den oberen Bereichen der Längsseitenwände 5, 6, 7 8 eingelassenen Ankerelementen 34 verbunden sind. An den längeren Fermenterbehältern 2, 3 sind jeweils zwei Kreuzstrebenanordnungen 33 nebeneinander verwendet, während im kürzeren Fermenterbehälter 4 eine Kreuzstrebenanordnung 33 ausreichend ist. Solche an sich bekannten Kreuzstrebenabstützungen nehmen Druck und Zugbelastungen auf und sind im vorliegenden Fall für eine statische Aussteifung gut geeignet.

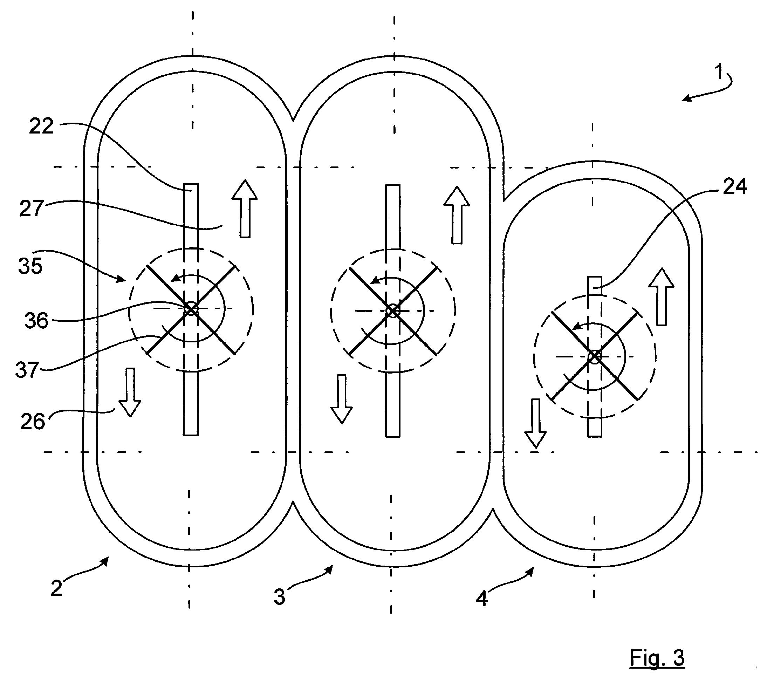

In Fig. 3 ist ein Fermenterverbund 1 aus Fermenterbehältern 2, 3, 4 entsprechend der Anordnung von Fig. 1 gezeigt, jedoch mit anderen Rühreinrichtungen. In den Fermenterbehältern 2, 3, 4 ist jeweils ein Paddelrührwerk 35 mit vertikaler Rührwelle 36 eingesetzt, wobei an der Rührwelle 36 winkelversetzt Rührpaddel 37 oder Rührflügel angeordnet sind. Die Rührwelle ist jeweils im Bereich der Längsmitte der Mittenwände 22, 23, 24 angeordnet, wo diese zum Durchgang der Rührpaddel 37 Ausnehmungen aufweisen. Bei dieser Anordnung ist eine Schubrichtung der Rührpaddel bei der eingezeichneten Drehrichtung jeweils im Längskanal 26 entsprechend dem dort eingezeichneten Strömungspfeil und eine gegensinnige Schubrichtung im anderen Längskanal 27 entsprechend dem dort gegensinnig gerichteten Strömungspfeil. Damit ergibt sich ein effektives Rühr- und Mischergebnis mit umlaufender Strömung bei relativ geringem Energieaufwand.

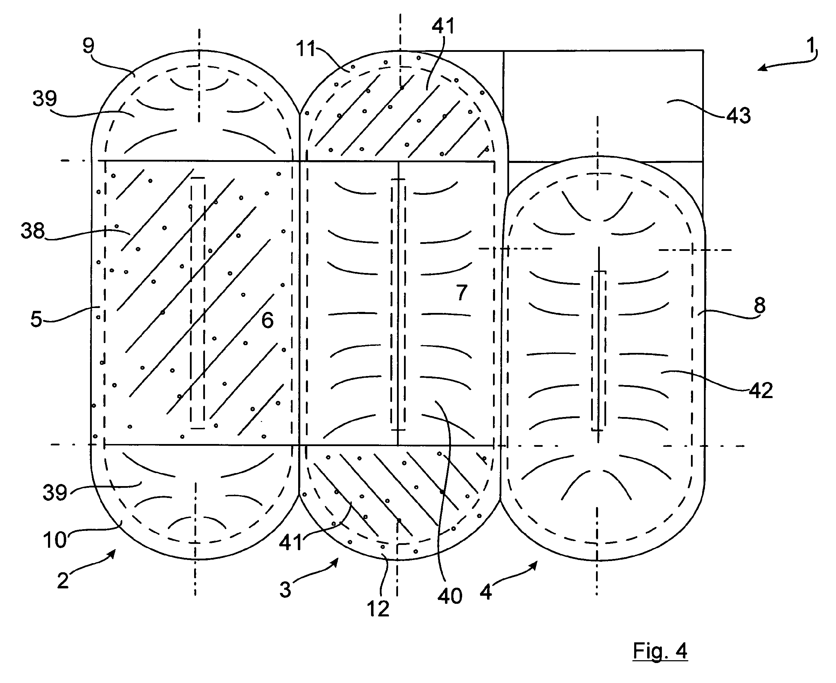

In Fig. 4 ist wiederum eine Draufsicht auf einen Fermenterverbund 1 entsprechend Fig. 1 gezeigt mit den Fermenterbehältern 2, 3, 4, wobei in Fig. 4 unterschiedliche gasdichte Abdeckungen vergleichsweise und beispielhaft dargestellt sind.

Der Fermenterbehälter 2 ist hier im Bereich der Längsseitenwände 5, 6 durch eine Betondecke 38 abgedeckt, welche zudem den statischen Zusammenhalt der statisch hochbelasteten Längsseitenwände 5, 6 gewährleistet. Im Bereich der statisch weniger kritischen Bogenwände 9, 10 ist jeweils eine Abdeckung durch ein ballonförmiges Foliendach 39 durchgeführt.

Grundsätzlich ist auch die umgekehrte Kombination möglich, wie dies beispielhaft am Fermenterbehälter 3 gezeigt ist: Hier ist im Bereich der Längsseitenwände 6, 7 ein giebeldachförmiges Foliendach 40 angebracht, welches durch die einfache Form einfach herstellbar ist. Zur statischen Aussteifung können hier die Kreuzstrebenanordnungen gemäß Fig. 2 verwendet werden. Der restliche Bereich bis zu den Bogenwänden 11, 12 ist jeweils durch eine halbkreisförmige Betondecke 41 abgedeckt.

Der Fermenterbehälter 4 ist dagegen beispielhaft insgesamt durch ein Foliendach 42 abgedeckt, welches ebenso wie die Foliendächer bei den Fermenterbehältern 2, 3 zusätzlich als Gasspeicher verwendbar ist.

Die Fermenterbehälter 2, 3, 4 fluchten an einer Seite mit ihren Breitseiten. Da der Fermenterbehälter 4 jedoch kürzer ist, entsteht bezüglich der Fermenterbehälter 2, 3 ein Freiraum, der in Fig. 4 zur Integration eines Technikgebäudes 43 für beispielsweise Pumpen Steuerungen etc. genutzt ist.

## Patentansprüche

1. Biogasanlage zur Herstellung von Biogas durch anaerobe Nassvergärung eines Biomasse enthaltenden Substrats in einem einstufigen Durchflussverfahren,
mit wenigstens zwei Fermenterbehältern (2, 3, 4), insbesondere mit wenigstens einem Hauptfermenter, wenigstens einem Nachfermenter und wenigstens einem Endlager,
**dadurch gekennzeichnet,**
**dass** jeder der Fermenterbehälter (2, 3, 4) in der Art einer langgestreckten, überdachten Fermenterwanne aus einer Bodenwand und aus zwei parallelen, beabstandeten, vertikalen und geraden Längsseitenwänden (5 bis 8) besteht, die endseitig an der Breitseite der Fermenterbehälter (2, 3, 4) durch vertikale, konvex ausgeformte Breitseitenwände (9 bis 14) verbunden sind, und
**dass** wenigstens zwei Fermenterbehälter (2, 3, 4) in einem Fermenterverbund (1) mit Längsseitenbereichen zumindest teilweise nebeneinanderliegend aneinandergrenzen und durch ein gemeinsamer Längsseitenwandbereich als Verbundwand (6, 7) gebildet ist.

2. Biogasanlage nach Anspruch 1, **dadurch gekennzeichnet, dass** in den Fermenterbehältern (2, 3, 4) in der Längsmitte verlaufende, vertikale Mittenwände (22, 23, 24) angeordnet sind, die mit ihren Mittenwandlängsenden beabstandet (Pfeil 25) zu den benachbarten konvex ausgeformten Breitseitenwänden (9 bis 14) liegen, sodass in den Fermenterbehältern jeweils zwei parallel verlaufende, langgestreckte gegensinnig durchstörmbare Längskanäle (26, 27) mit endseitigen Kanalumlenkungen durch die Breitseitenwände (9 bis 14) zu einer Endlos-Kanalstruktur gebildet sind.

3. Biogasanlage nach Anspruch 1 oder Anspruch 2, **dadurch gekennzeichnet, dass** die Breitseitenwände halbkreisförmig kontinuierlich gebogene oder durch gerade Teilstücke bogenförmig zusammengesetzte Bogenwände (9 bis 14) sind, und
dass der Längsabstand (Pfeil 25) der Mittenwandlängsenden zu den benachbarten Bogenwänden (9 bis 14) größer oder zumindest gleich der Quererstreckung (Pfeil 28) eines Längskanals (26, 27) ist.

4. Biogasanlage nach einem der Ansprüche1 bis 3, **dadurch gekennzeichnet, dass** die Längsseitenwände (5 bis 8) und/oder Breitseitenwände (9 bis 14) zumindest teilweise aus Fertigteilelementen (17, 18, 19) zusammengesetzt sind, wobei insbesondere der Übergangsbereich zwischen einer gemeinsamen Verbundwand (6, 7) und einer anschlie-ßenden, einem einzelnen Fermenterbehälter (2, 3, 4) zugeordneten Wand aus einem Fertigteil (17, 18) gebildet ist.

5. Biogasanlage nach einem der Ansprüche 1 bis 4, **dadurch gekennzeichnet, dass** die Längsseitenwände (5 bis 8) und/oder die Breitseitenwände (9 bis 14) und/oder die Mittenwände (22, 23, 24) aus Beton und/oder Stahl und/oder Kunststoff und/oder Holz hergestellt sind, wobei zumindest die Außenwände des Fermenterverbunds (1) statisch sicher ausgebildet sind.

6. Biogasanlage nach Anspruch 5, **dadurch gekennzeichnet, dass** fermenterverbundinnere Behälterzwischenwände (6, 7) und/oder Mittenwände (22, 23, 24) als Tauchwände in der Art gespannter Textilfahnen und/oder Kunststoffbahnen und/oder Holzwände ausgeführt sind.

7. Biogasanlage nach einem der Ansprüche 1 bis 6, **dadurch gekennzeichnet, dass** die Längsseitenwände (5 bis 8) eines Fermenterbehälters (2, 3, 4), jedoch zumindest die äußeren Längsseitenwände (5, 8) des Fermenterverbunds (1) im oberen Bereich durch Kreuzstreben (33) zur statischen Aussteifung verbunden sind.

8. Biogasanlage nach einem der Ansprüche 1 bis 7, **dadurch gekennzeichnet, dass** der Fermenterverbund (1) gemeinsam oder einzelne Fermenterbehälter (2, 3, 4) separat durch eine Betondecke und/oder durch Stahlplatten und/oder durch ein Foliendach gasdicht abgedeckt sind.

9. Biogasanlage nach Anspruch 8, **dadurch gekennzeichnet, dass** die Bereiche der Längsseitenwände (5 bis 8) durch eine Betondecke (38) und die Bereiche der konvex ausgeformten Breitseitenwände insbesondere der Bogenwände (9 bis 14) jeweils durch ein Foliendach (39) abgedeckt sind.

10. Biogasanlage nach Anspruch 8 oder 9, **dadurch gekennzeichnet, dass** in den Betondecken- und/oder Foliendachbereichen Revisionsöffnungen, insbesondere Revisionsschächte (30) und/oder Sichtfenster (32) angeordnet sind.

11. Biogasanlage nach einem der Ansprüche 1 bis 10, **dadurch gekennzeichnet, dass** in den Fermenterbehältern (2, 3, 4) vorzugsweise in den Längskanälen (26, 27) wenigstens eine Rühreinrichtung (29, 30, 35) angeordnet ist, mit der das Substrat im zugeordneten Fermenterbehälter (2, 3, 4) in eine umlaufende Bewegung versetzbar ist.

12. Biogasanlage nach Anspruch 11, **dadurch gekennzeichnet, dass** die wenigstens eine Rühreinrichtung ein Tauchrührgerät (29) umfasst ist, das einen elektrisch oder hydraulisch antriebbaren Motor aufweist und das höhenverstellbar durch einen Revisionsschacht (30) im Fermenterbehälter (2, 3, 4) oder an einem Brückensteg angeordnet ist, und/oder
dass die Rühreinrichtung ein seitlich durch eine Außenwand des Fermenterverbunds einführbares Rührwerk (31) umfasst, und/oder
dass das Rührwerk ein Paddelrührwerk (35) mit vertikaler Rührwelle (36) ist, die im Bereich einer Mittenwand (22, 23, 24), vorzugsweise im Bereich deren Längsmitte angeordnet ist, sodass die Rührpaddel (37) mit einer Schubrichtung in einem Längskanal (26) und mit der gegensinnigen Schubrichtung im anderen parallelen Längskanal (27) drehend eingreifen.

13. Biogasanlage nach einem der Ansprüche 1 bis 12, **dadurch gekennzeichnet, dass** zumindest ein Fermenterbehälter (4) des Fermenterverbunds (1) eine vergleichsweise geringere Längserstreckung aufweist und in dem **dadurch** geschaffenen Freiraum ein Technikgebäude (43), insbesondere für Pumpen und/oder für Steuerungen und/oder für ein Blockheizkraftwerk (BHKW), oder ein Gasspeicherraum in den Fermenterverbund integriert ist.

14. Biogasanlage nach einem der Ansprüche 1 bis 13, **dadurch gekennzeichnet, dass** am Fermenterverbund (1) wenigstens eine Zudosieranlage für flüssige und/oder feste Substratbestandteile angeordnet ist, und
dass Leitungsverbindungen zwischen den Fermenterbehältern (2, 3, 4), insbesondere Überläufe jeweils unmittelbar im Bereich einer Verbundwand (6, 7) angebracht sind.
